# EUROPEAN PATENT SPECIFICATION

(11) **EP 3 393 567 B1**
(45) Date of publication and mention of the grant of the patent: **30.06.2021**
(21) Application number: 16806177.8
(22) Date of filing: 08.12.2016
(51) Int. Cl.: A61M 15/06, A24F 47/00

(54) **AEROSOL-GENERATING SYSTEM WITH PUMP**
AEROSOL-ERZEUGUNGSSYSTEM MIT PUMPE
SYSTÈME DE GÉNÉRATION D'AÉROSOL AVEC POMPE

(30) Priority: 22.12.2015 EP 15202158
(43) Date of publication of application: 31.10.2018
(73) Proprietor: Philip Morris Products S.A., 2000 Neuchâtel (CH)
(72) Inventor: PHILLIPS, Shaun, Peterborough Cambridgeshire PE7 1JH (GB); RENFREW, Bruce, Leicester Leicestershire LE2 8PH (GB); MAZUR, Ben, Bristol BS7 9QP (GB); BRIGHT, Ben, Tewkesbury Gloucestershire GL20 6FL (GB); BATISTA, Rui Nuno, 1110 Morges (CH)
(74) Representative: Ponder, William Anthony John
(86) International application number: PCT/EP2016/080245
(87) International publication number: WO 2017/108429

(56) References cited:
- EP-A1- 2 047 880
- US-A1- 2002 078 946
- US-A1- 2003 136 404
- US-A1- 2014 060 554
- US-A1- 2014 353 856
- US-A1- 2015 027 456
- US-A1- 2015 216 237

## Description

The present invention relates to aerosol-generating systems, such as handheld electrically operated smoking systems. In particular, the present invention relates to aerosol-generating systems in which the aerosol-forming substrate is liquid and is contained in a liquid storage portion.

One type of aerosol-generating system is an electrically operated smoking system. Handheld electrically operated smoking systems are known that consist of a device portion comprising a battery and control electronics, a cartridge portion comprising a supply of aerosol-forming substrate held in a liquid storage portion, and an electrically operated vaporiser. A cartridge comprising both a supply of aerosol-forming substrate held in the liquid storage portion and a vaporiser is sometimes referred to as a "cartomiser". The vaporiser typically comprises a coil of heater wire wound around an elongate wick soaked in the liquid aerosol-forming substrate held in the liquid storage portion. The cartridge portion typically comprises not only the supply of aerosol-forming substrate and an electrically operated vaporiser, but also a mouthpiece, which the user sucks on in use to draw aerosol into their mouth.

US 2015/0216237 A1 discloses methods and devices for transitioning a smoker to an electronic nicotine delivery device and for smoking or nicotine urge relief. One such device comprises a reservoir comprising a liquid formulation comprising a pharmaceutically active agent, and a pump, wherein the pump is located within the reservoir, and wherein the pump is in fluid communication with the liquid formulation. The device further comprises a heater element, wherein the heater element is in fluid communication with the pump, and wherein the pump is configured to deliver the liquid formulation to the heater element. The heater element is configured to vaporize the liquid formulation upon activation to generate a condensation aerosol.

US 2015/0027456 A1 discloses an electronic smoking article including an aerosol generator and a mouth end insert.

US 2003/136404 A1 discloses an aerosol generator including a housing, a heater and a mouthpiece, wherein the heater volatilizes liquid material within a flow passage and forms an aerosol in the mouthpiece. The aerosol generator may comprise a syringe pump that can be operated to deliver a predetermined volume of liquid material from the liquid source to the flow passage. In some embodiments, the aerosol generator may comprise an airflow entrainment control member having a conical configuration.

EP 0 957 959 B1 discloses an electrically operated aerosol generator for receiving liquid material from a source, the aerosol generator comprising a pump for pumping the liquid material in metered amounts from the source through a tube with an open end, and a heater surrounding the tube. When heating the liquid material by the heater, the volatized material expands by exiting the open end of the tube.

Residues are created upon heating. In capillary tubes, the residues can cause clogging. This effect can alter liquid transport properties. Furthermore, the liquid material is heated indirectly: First the tube or a capillary wick is heated which in turn heats the liquid material. Heat can therefore be lost during the energy transfer process.

It would be desirable to provide an improved aerosol-generating system with a low-maintenance liquid transport system and reduced power consumption.

The scope of the invention is as defined by the appended claims.

According to a first aspect there is provided an aerosol-generating system comprising a liquid storage portion for storing liquid aerosol-forming substrate, a vaporiser comprising a heating element having a structure defining an open-ended internal passage, and a micro pump configured for delivering liquid aerosol-forming substrate from the liquid storage portion to the internal passage of the heating element, wherein the vaporiser is configured for heating the delivered liquid aerosol-forming substrate at the internal passage to a temperature sufficient to volatilize at least a part of the delivered liquid aerosol-forming substrate.

A determined amount of liquid aerosol-forming substrate is pumped from the liquid storage portion to the internal passage of the heating element. By depositing the liquid aerosol-forming substrate to the heating element directly, the liquid aerosol-forming substrate can remain in its liquid state until it reaches the heating element. Consequently, the production of residues can be minimized during liquid transport. Such a design can allow the production of cartridges without vaporisers. Due to the improved liquid transport, tubing segments and vaporisers may not need to be disposed once the liquid storage portion is empty. By using a pump instead of a capillary wick or any other passive medium to draw liquid, only the actually required amount of liquid aerosol-forming substrate may be transported to the heating element. Liquid aerosol-forming substrate may only be pumped upon demand, for example in response to a puff by a user.

The micro pump may allow on-demand delivery of liquid aerosol-forming substrate at a low flow rate of for example approximately 0.5 to 2 microlitres per second for intervals of variable or constant duration. The micro pump can be carefully tuned in order to deliver the appropriate amount of liquid aerosol-forming substrate to the heating element. Consequently, the amount of deposited liquid aerosol-forming substrate can be determined from the amount of pump cycles.

The micro pump may be configured to pump liquid aerosol-forming substrates that are characterized by a relatively high viscosity as compared to water. The viscosity of a liquid aerosol-forming substrate may be in the range from about 10 to 500 Millipascalseconds, preferably in the range from about 17 to 86 Millipascalseconds.

The micro pump may be a single micro pump, for example comprising a pump inlet, a pump outlet, and a pump chamber between the pump inlet and the pump outlet. Liquid aerosol-forming substrate to be pumped is available at the pump inlet. The single micro pump may be based on a piezoelectric diaphragm in combination with a passive check valve at each of the pump inlet and the pump outlet. A piezo ceramic mounted on a membrane may be deformed upon applying a voltage. The resulting down stroke causes liquid aerosol-forming substrate to be displaced out of the pump chamber. The check valve can ensure against backflow between usages, for example into the cartridge. The pump controls the flow direction of the liquid through the pump from the inlet to the outlet. When the voltage applied to the piezo ceramic decreases, the corresponding piezo deformation causes an upstroke of the membrane. Liquid aerosol-forming substrate is sucked from the pump inlet and fills the pump chamber. The single micro pump may perform up to several hundreds of such pump cycles per second, for example up 100 pump cycles per second, 200 pump cycles per second, 300 pump cycles per second, 400 pump cycles per second, or 500 pump cycles per second.

The micro pump may comprise a plurality of single micro pumps in series where the pump outlet of a single micro pump is connected to the pump inlet of the following single micro pump. Preferably, the micro pump comprises two single micro pumps in series. To ensure a proper flow of liquid through the series of single micro pumps, the piezo transducers can for example be operated sequentially after each other. In case of two single micro pumps, the piezo transducer corresponding to the first single micro pump is actuated to perform an up and down stroke, and then the piezo transducer corresponding to the second single micro pump is actuated to perform an up and down stroke. One pump cycle of a micro pump with a plurality of single micro pumps may require actuation of the piezo transducers of one single micro pump after the other.

When adjusting the flow rate, more energy may be required to vaporise the higher amount of delivered liquid aerosol-forming substrate at the heating element. Therefore, the temperature settings of the vaporiser may be adjusted in accordance to the liquid flow rate.

The temperature of the heating element is preferably controlled by electric circuitry. Preferably, the electric circuitry controls the temperature of the heating element based on at least one of a preheat duration parameter, a hold high duration parameter, a hold low duration parameter, and a hold timeout duration parameter, as explained in detail below.

The aerosol-generating system may be in a preheat mode when the aerosol-generating has been powered on. The electric circuitry preferably heats the heating element constantly until the heating element reaches a predetermined operating temperature. The operating temperature may be 250 °C. The duration of the preheat mode may be controlled by the preheat duration parameter. Preferably, the preheat duration parameter is set to a value between 3000 and 4000 milliseconds. Under normal conditions, the aerosol-generating system may reach its operating temperature within the preheat duration. Once the operating temperature is reached, the micro pump may be activated for at least one pump cycle to transport an amount of liquid aerosol-forming substrate through the micro pump to make the aerosol-generating system ready to use.

The aerosol-generating system may then enter the temperature hold mode until the electric circuitry detects a puff or until a hold timeout duration is reached as defined by the value of the hold timeout duration parameter.

In temperature hold mode, a sequence of heating pulses may be sent to the heating element which can have the effect of maintaining a lower preheat temperature below the operating temperature until the hold timeout is reached. The lower preheat temperature may be 150 °C.

A heating pulse may comprise a sequence of a high and a low signal, for example wherein the high signal causes applying power to the heating element for the value of the hold high duration parameter, and wherein the low signal turns off the power applied to the heating element for the duration of the value of the hold low duration parameter. The duration of the temperature hold mode may be controlled by the value of the hold timeout duration parameter, before the aerosol-generating system enters a cool-down mode.

The practical effect of these different modes can be that there is reduced latency on puffing as the heating element is already hot during regular puffing, but the aerosol-generating system may save power when turned on but not in use. When input is detected from the puff sensor, the alternating signal of the sequence of heating pulses may be replaced by a constant signal to bring the temperature of the heating element back to the operating temperature.

Once a puff has been detected and the heating element has reached the operating temperature, the electric circuitry may activate the micro pump and set a determined flow rate for delivering liquid aerosol-forming substrate to the internal passage of the heating element for the duration of the puff.

Once the puff stops, the aerosol-generating system may return to the temperature hold mode. If no input is detected during the temperature hold duration, the device may automatically cool down and wait for input.

Both the micro pump and the heating element may be triggered by a puff detection system. Alternatively, the micro pump and the heating element may be triggered by pressing an on-off button, held for the duration of a puff.

Preferably, the micro pump is configured to deliver a determined amount of liquid aerosol-forming substrate from the liquid storage portion to the internal passage of the heating element upon performing one pump cycle.

Preferably, the aerosol-generating system further comprises a chamber into which the liquid aerosol-forming substrate is delivered, and wherein the heating element is arranged inside the chamber downstream of an outlet of the micro pump through which the liquid aerosol-forming substrate is delivered.

As used herein, the terms 'upstream', 'downstream', 'proximal', 'distal', 'front' and 'rear', are used to describe the relative positions of components, or portions of components, of the aerosol-generating system in relation to the direction in which a user draws on the aerosol-generating system during use thereof.

The aerosol-generating system may comprise a mouth end through which in use an aerosol exits the aerosol-generating system and is delivered to a user. The mouth end may also be referred to as the proximal end. In use, a user draws on the proximal or mouth end of the aerosol-generating system in order to inhale an aerosol generated by the aerosol-generating system. The aerosol-generating system comprises a distal end opposed to the proximal or mouth end. The proximal or mouth end of the aerosol-generating system may also be referred to as the downstream end and the distal end of the aerosol-generating system may also be referred to as the upstream end. Components, or portions of components, of the aerosol-generating system may be described as being upstream or downstream of one another based on their relative positions between the proximal, downstream or mouth end and the distal or upstream end of the aerosol-generating system.

The aerosol-generating system further comprises a tubing segment through which the liquid aerosol-forming substrate is delivered from the micro pump to the vaporiser. The tubing segment may be arranged to deliver the liquid aerosol-forming substrate directly to the heating element. The tubing segment may be arranged to deliver the liquid aerosol-forming substrate towards an open end of the internal passage in the heating element. The tubing segment may extend from the liquid storage portion in a direction towards an open end of the internal passage in the heating element. The vaporiser may be arranged downstream of an open end of the tubing segment. The vaporiser may extend around a portion of the tubing segment.

The tubing segment, also referred to as tube, may be a nozzle. The tubing segment may comprise any appropriate material, for example glass, silicon, metal, for example stainless steel, or plastics material, for example PEEK. The size of the tube may match that of the pump outlet. For example, the tube may have a diameter of about 1 to 2 millimetres but other sizes are possible. The tubing segment may be a 2 millilitres capillary nozzle (for example glass from Drummond) connected to the micro pump via a silicon tubing. Preferably, the tubing segment comprises a capillary tube. The cross-section of the capillary tube may be circular, ellipsoid, triangular, rectangular or any other suitable shape to convey liquid. At least a width dimension of the cross-sectional area of the capillary tube preferably may be chosen to be sufficiently small such that on the one hand capillary forces are present. At the same time, the cross-sectional area of the capillary tube needs is preferably sufficiently large such that a suitable amount of liquid aerosol-forming substrate can be conveyed to the heating element. In general, the cross-sectional area of the capillary tube is in some examples below 4 square millimetres, below 1 square millimetre, or below 0.5 square millimetres.

The vaporiser may comprise a heating coil extending from the tubing segment in longitudinal direction. Alternatively, or in addition, the heating element, which may be a coil, may extend around a portion of the tubing segment. The heating coil may be mounted transverse to the tubing segment. The heating coil may overlap with the open end of the tubing segment for up to 3 millimetres, preferably for up to 1 millimetre. In some examples, there may be a distance between the open end of the tubing segment and the heating coil. The length of the heating coil may be 2 millimetres to 9 millimetres, preferably 3 millimetres to 6 millimetres. The diameter of the heating coil may be chosen such that one end of the heating coil can be mounted around the tubing segment. The diameter of the heating coil may be 1 millimetre to 5 millimetres, preferably 2 millimetres to 4 millimetres.

The vaporiser comprises a conical heater extending from the tubing segment in longitudinal direction. The conical heater may overlap with the open end of the tubing segment. In some examples, there may be a distance of 0.1 millimetres to 2 millimetres between the open end of the tubing segment and the conical heater, preferably 0.1 millimetres to 1 millimetre. The slant height of the conical heater may be 2 millimetres to 7 millimetres, preferably 2.5 millimetres to 5 millimetres. The diameter of the conical heater in cross-sectional view may increase, when following the slant height from one end to the other, from a first diameter to a second diameter. The first diameter may be 0.1 millimetres to 2 millimetres, preferably 0.1 millimetres to 1 millimetre. The second diameter may be 1.2 millimetres to 3 millimetres, preferably 1.5 millimetres to 2 millimetres. Preferably, the conical heater is arranged such that the liquid aerosol-forming substrate exiting from the tubing segment passes the conical heater at the first diameter before the second diameter. The first diameter of the conical heater may be chosen such that one end of the conical heater can be mounted around the tubing segment.

The vaporiser may comprise a solid or mesh surface. The vaporiser may comprise a mesh heater. The vaporiser may comprise an arrangement of filaments.

The vaporiser may comprise at least one of a solid, flexible, porous, and perforated substrate onto which the heating element may be for example at least one of mounted, printed, deposited, etched, and laminated. The substrate may be a polymeric or ceramic substrate.

Preferably, the liquid storage portion comprises a rigid container and a one-way valve that may be configured to let air into the container upon delivering liquid aerosol-forming substrate from the liquid storage portion to the internal passage of the heating element.

Preferably, the liquid storage portion comprises a flexible container with at least one movable wall that may be configured to reduce the volume of the liquid storage portion upon delivering liquid aerosol-forming substrate from the liquid storage portion to the internal passage of the heating element.

Preferably, the liquid storage portion comprises a septum for refilling the liquid storage portion with liquid aerosol-forming substrate.

Preferably, the liquid storage portion comprises a one-way valve arranged between the liquid storage portion and the micro pump. The one-way valve may control the liquid flow from the liquid storage portion to the inlet of the micro pump and may avoid or reduce reflux back to the liquid storage portion once the micro pump stops pumping.

Preferably, the flow rate of the delivered liquid aerosol-forming substrate through the micro pump is within 0.5 to 2 microlitres per second.

Preferably, the aerosol-generating system comprises a main unit and a cartridge, wherein the cartridge may be removably coupled to the main unit, wherein the main unit may comprise a power supply, wherein the liquid storage portion may be provided in the cartridge, and wherein the micro pump may be provided in the main unit. Preferably, the main unit further comprises the vaporiser. The main unit may comprise the tubing segment.

The aerosol-generating system according to an embodiment of the present invention may further comprise electric circuitry connected to the vaporiser and to an electrical power source. The electric circuitry may be configured to monitor the electrical resistance of the vaporiser, and preferably to control the supply of power to the vaporiser dependent on the electrical resistance of the vaporiser.

The electric circuitry may comprise a controller with a microprocessor, which may be a programmable microprocessor. The electric circuitry may comprise further electronic components. The electric circuitry may be configured to regulate a supply of power to the vaporiser. Power may be supplied to the vaporiser continuously following activation of the system or may be supplied intermittently, such as on a puff-by-puff basis. The power may be supplied to the vaporiser in the form of pulses of electrical current.

The aerosol-generating system advantageously comprises a power supply, typically a battery, for example within the main body of the housing. The power supply may be a form of charge storage device such as a capacitor. The power supply may require recharging and may have a capacity that allows for the storage of enough energy for one or more smoking experiences; for example, the power supply may have sufficient capacity to allow for the continuous generation of aerosol for a period of around six minutes or for a period that is a multiple of six minutes. In another example, the power supply may have sufficient capacity to allow for a predetermined number of puffs or discrete activations of the heater assembly.

For allowing ambient air to enter the aerosol-generating system, a wall of the housing of the aerosol-generating system, preferably a wall opposite the vaporiser, preferably a bottom wall, is provided with at least one semi-open inlet. The semi-open inlet preferably allows air to enter the aerosol-generating system, but no air or liquid to leave the aerosol-generating system through the semi-open inlet. A semi-open inlet may for example be a semi-permeable membrane, permeable in one direction only for air, but is air- and liquid-tight in the opposite direction. A semi-open inlet may for example also be a one-way valve. Preferably, the semi-open inlets allow air to pass through the inlet only if specific conditions are met, for example a minimum depression in the aerosol-generating system or a volume of air passing through the valve or membrane.

The liquid aerosol-forming substrate is a substrate capable of releasing volatile compounds that can form an aerosol. The volatile compounds may be released by heating the liquid aerosol-forming substrate. The liquid aerosol-forming substrate may comprise plant-based material. The liquid aerosol-forming substrate may comprise tobacco. The liquid aerosol-forming substrate may comprise a tobacco-containing material containing volatile tobacco flavour compounds, which are released from the liquid aerosol-forming substrate upon heating. The liquid aerosol-forming substrate may alternatively comprise a non-tobacco-containing material. The liquid aerosol-forming substrate may comprise homogenised plant-based material. The liquid aerosol-forming substrate may comprise homogenised tobacco material. The liquid aerosol-forming substrate may comprise at least one aerosol-former. The liquid aerosol-forming substrate may comprise other additives and ingredients, such as flavourants.

The aerosol-generating system may be an electrically operated smoking system. Preferably, the aerosol-generating system is portable. The aerosol-generating system may have a size comparable to a conventional cigar or cigarette. The smoking system may have a total length between approximately 30 millimetres and approximately 150 millimetres. The smoking system may have an external diameter between approximately 5 millimetres and approximately 30 millimetres.

According to a second aspect of the present invention there is provided a cartridge for the aerosol-generating system according to the first aspect of the present invention, wherein the cartridge comprises the liquid storage portion. The liquid storage portion comprises an outlet that is configured to connect to the inlet of the micro pump.

Preferably, the cartridge comprises a cover that covers the outlet of the liquid storage portion. The cover may be a pulled sticker or a seal, for example a film seal, which may protect the cartridge before use. The cover could be removed from the cartridge by hand before inserting the cartridge into the main unit. Preferably, the cover is punctured or pierced so that the cover opens automatically upon inserting the cartridge into the main unit.

The cartridge may be a disposable article to be replaced with a new cartridge once the liquid storage portion of the cartridge is empty or below a minimum volume threshold. Preferably, the cartridge is pre-loaded with liquid aerosol-forming substrate. The cartridge may be refillable.

The cartridge and its components may be made of thermoplastic polymers, such as polyether ether ketone (PEEK).

According to a third aspect of the present invention there is provided a method for generating aerosol, comprising:
(i) storing liquid aerosol-forming substrate in a liquid storage portion,
(ii) delivering, by a micro pump, liquid aerosol-forming substrate from the liquid storage portion to an internal passage within a heating element of a vaporiser, and
(iii) heating, by the vaporiser, the delivered liquid aerosol-forming substrate in the internal passage to a temperature sufficient to volatilize at least a part of the delivered liquid aerosol-forming substrate.

Features described in relation to one aspect may equally be applied to other aspects of the invention.

Embodiments of the invention will now be described, by way of example only, with reference to the accompanying drawings, in which:
Figure 1A is a perspective and a topside view of an aerosol-generating system;
Figure 1B is a perspective view of an aerosol-generating system;
Figure 2 is a topside view of a tubing segment and a heating coil for an aerosol-generating system;
Figure 3A is a topside view of a tubing segment and a conical heater for an aerosol-generating system in accordance with an embodiment of the present invention;
Figure 3B is schematic illustration for making the conical heater shown in Figure 3A;
Figure 4 is a topside view of a refillable cartridge for an aerosol-generating system;
Figure 5 is a topside view of a refillable cartridge for an aerosol-generating system; and
Figure 6 is a topside view of a refillable cartridge for an aerosol-generating system.

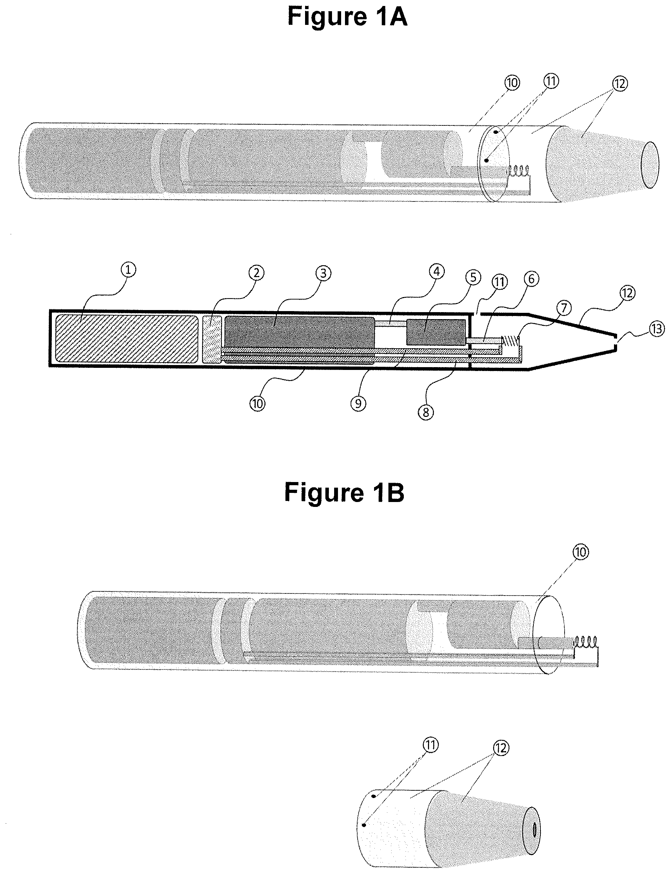

Figure 1A and 1B are schematic illustrations of an aerosol-generating system. The aerosol-generating system comprises a main unit and a refillable or exchangeable cartridge with a liquid storage portion 3. The main unit comprises a main body 10 and a mouthpiece portion 12. The main body 10 contains a power supply 1, for example a battery such as a lithium iron phosphate battery, electronic circuitry 2, a cavity for holding a cartridge, a micro pump 5 with an inlet and an outlet, and a vaporiser 7. Electrical connectors 8, 9 are provided at the sides of the main body 10 to provide an electrical connection between the electric circuitry 2, the power supply 1, and the vaporiser 7. The vaporiser 7 is a heating coil defining an internal passage. Tubing segment 4 is provided to connect an outlet of the liquid storage portion to the inlet of the micro pump. Tubing segment 6 leads the flow of liquid aerosol-forming substrate from the outlet of the micro pump 5 to the internal passage of the heating element of the vaporiser 7. The tubing segment 6 is straight and transports the liquid aerosol-forming substrate directly to the heating element. The mouthpiece portion 12 comprises a plurality of air inlets 11 and an outlet 13. In use, a user sucks or puffs on the outlet 13 to draw air from the air inlets 11, through the mouthpiece portion 12 to the outlet 13, and thereafter into the mouth or lungs of the user. Internal baffles are provided to force the air flowing through the mouthpiece portion 12. The vaporiser 7 is configured to heat the liquid aerosol-forming substrate directly after the liquid aerosol-forming substrate exits the tubing segment 6.

The cartridge is configured to be received in a cavity within the main body 10. The cartridge should be replaceable by a user when the aerosol-forming substrate provided in the cartridge is depleted. When inserting a new cartridge, a slider at the main body may be moved to expose the cavity. A new cartridge may be inserted into the exposed cavity. The outlet of the liquid storage portion is configured to connect to the inlet of the micro pump 5. The main unit is portable and has a size comparable to a conventional cigar or cigarette.

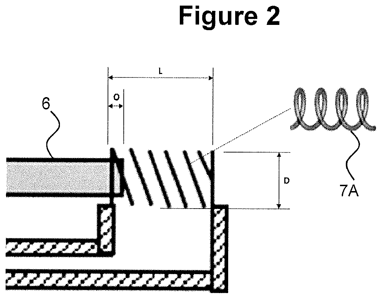

Figure 2 shows a detail of the open ended side of the tubing segment 6. A heating coil 7A is mounted onto the open ended side of the tubing segment 6 such that the heating coil 7A extends from the tubing segment 6 in longitudinal direction. Liquid aerosol-forming substrate exits at the open end of the tubing segment 6. The liquid aerosol-forming substrate exits the tubing segment 6 into the internal passage of the coil 7A.The heating coil 7A is in and around the flow of liquid so that the liquid aerosol-forming substrate is directly heated. The heating coil 7A has a length L, a diameter D, and an overlap O with the tubing segment 6.

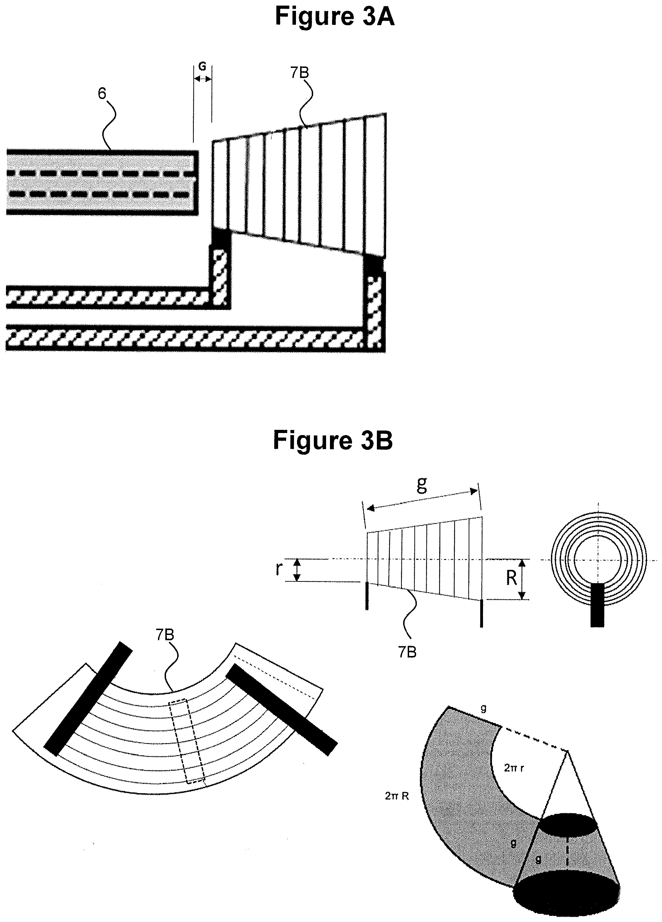

Figure 3A shows a detail of the open ended side of the tubing segment 6. A conical heater 7B is mounted downstream the open ended side of the tubing segment 6 such that the conical heater 7B extends from the tubing segment 6 in longitudinal direction. Liquid aerosol-forming substrate exits at the open end of the tubing segment 6 into an internal passage defined by the conical heater 7B. The conical heater 7B is in and around the flow of liquid so that the liquid aerosol-forming substrate is directly heated. There is a distance G between the cone end side of the conical heater 7B and the tubing segment 6.

Figure 3B is a schematic illustration of making the conical heater 7B from a flat substrate. The conical heater 7B has a slant height g with a radius that increases from a first radius r to a second radius R.

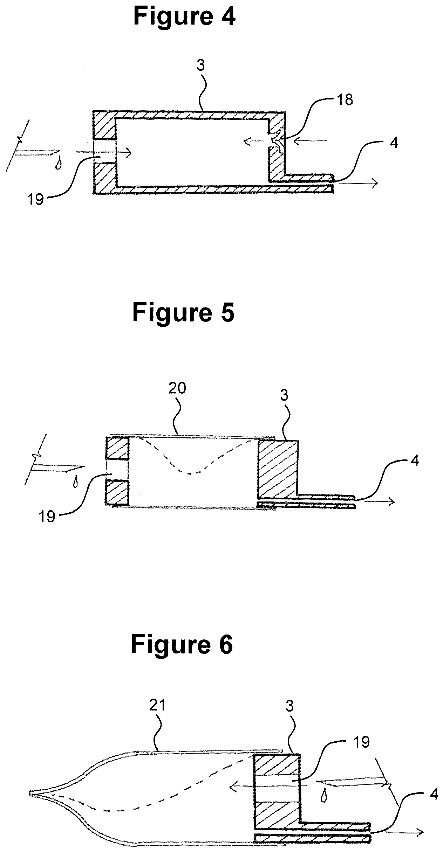

Figure 4 shows a refillable cartridge for an aerosol-generating system. The cartridge comprises a liquid storage portion 3 made of a rigid container. The container comprises an outlet connected via a tubing segment 4 to the inlet of the micro pump. Air is allowed to enter the rigid container via a one-way valve 18 upon pumping liquid. A septum 19 is provided for refilling the cartridge.

Figure 5 shows a refillable cartridge for an aerosol-generating system. The cartridge comprises a liquid storage portion 3 made of a rigid container with a flexible wall 20 made from a flexible tube. The container comprises an outlet connected via a tubing segment 4 to the inlet of the micro pump. Upon pumping liquid, the flexible wall 20 collapses. A septum 19 is provided at the rigid container for refilling the cartridge.

Figure 6 shows a refillable cartridge for an aerosol-generating system. The cartridge comprises a liquid storage portion 3 made of a rigid faceplate with two flexible walls 21 each made from a flexible tube. The container comprises an outlet connected via a tubing segment 4 to the inlet of the micro pump. Upon pumping liquid, the flexible walls 21 collapse. A septum 19 is provided at the rigid faceplate for refilling the cartridge.

The exemplary embodiments described above illustrate but are not limiting. In view of the above discussed exemplary embodiments, other embodiments consistent with the above exemplary embodiments will now be apparent to one of ordinary skill in the art.

## Claims

1. An aerosol-generating system, the system comprising:
a liquid storage portion (3) for storing liquid aerosol-forming substrate;
a vaporiser (7) comprising a heating element having a structure defining an open-ended internal passage;
a micro pump (5) configured for delivering liquid aerosol-forming substrate from the liquid storage portion to the internal passage of the heating element; and
a tubing segment (6) through which the liquid aerosol-forming substrate is delivered from the micro pump to the vaporiser;
wherein the heating element comprises a conical heating element extending from the tubing segment in a longitudinal direction and the vaporiser is configured for heating the delivered liquid aerosol-forming substrate at the internal passage to a temperature sufficient to volatilize at least a part of the delivered liquid aerosol-forming substrate.

2. An aerosol-generating system according to claim 1, wherein the micro pump (5) is configured to deliver a determined amount of liquid aerosol-forming substrate from the liquid storage portion (3) to the internal passage of the heating element upon performing one pump cycle.

3. An aerosol-generating system according to claim 1 or claim 2, wherein the system further comprises a chamber into which the liquid aerosol-forming substrate is delivered, and wherein the heating element is arranged inside the chamber downstream of an outlet of the micro pump through which the liquid aerosol-forming substrate is delivered.

4. An aerosol-generating system according to claim 1, wherein the vaporiser (7) is arranged downstream of an open end of the tubing segment (6).

5. An aerosol-generating system according to claim 1 or 4, wherein the tubing segment (6) comprises a capillary tube.

6. An aerosol-generating system according to claim 1, 4 or 5, wherein the vaporiser (7) comprises a heating coil extending around a portion of the tubing segment (6).

7. An aerosol-generating system according to claim 1, 4 or 5, wherein the vaporiser (7) comprises a heating coil extending from the tubing segment (6) in longitudinal direction.

8. An aerosol-generating system according to any of claims 1 to 7, wherein the liquid storage portion (3) comprises a rigid container and a one-way valve (18) that is configured to let air into the container upon delivering liquid aerosol-forming substrate from the liquid storage portion to the internal passage of the heating element.

9. An aerosol-generating system according to any of claims 1 to 7, wherein the liquid storage portion comprises a flexible container with at least one movable wall that is configured to reduce the volume of the liquid storage portion upon delivering liquid aerosol-forming substrate from the liquid storage portion to the internal passage of the heating element.

10. An aerosol-generating system according to any of claims 1 to 9, wherein the liquid storage portion (3) comprises a septum (19) for refilling the liquid storage portion with liquid aerosol-forming substrate.

11. An aerosol-generating system according to any of claims 1 to 10, wherein the liquid storage portion (3) comprises a one-way valve (18) arranged between the liquid storage portion and the micro pump (5).

12. An aerosol-generating system according to any of claims 1 to 11, wherein the flow rate of the delivered liquid aerosol-forming substrate through the micro pump (5) is within 0.5 to 2 microlitres per second.

13. An aerosol-generating system according to any of claims 1 to 12, wherein the system comprises a main unit and a cartridge, wherein the cartridge is removably coupled to the main unit, wherein the main unit comprises a power supply (1), wherein the liquid storage portion (3) is provided in the cartridge, and wherein the micro pump (5) is provided in the main unit.

14. A method for generating aerosol, comprising:
storing liquid aerosol-forming substrate in a liquid storage portion (3);
delivering, by a micro pump (5), liquid aerosol-forming substrate from the liquid storage portion, through a tubing segment (6), to an internal passage within a heating element of a vaporiser (7), the heating element comprising a conical heating element extending from the tubing segment in a longitudinal direction; and
heating, by the vaporiser, the delivered liquid aerosol-forming substrate in the internal passage to a temperature sufficient to volatilize at least a part of the delivered liquid aerosol-forming substrate.

## Patentansprüche

1. Aerosolerzeugungssystem, wobei das System aufweist:
ein Flüssigspeicherteil (3) zum Speichern von flüssigem aerosolbildendem Substrat;
einen Zerstäuber (7), der ein Heizelement mit einer Struktur aufweist, die einen offenen inneren Durchgang definiert;
eine Mikropumpe (5), die ausgelegt ist, um flüssiges aerosolbildendes Substrat von dem Flüssigspeicherteil an den inneren Durchgang des Heizelements abzugeben; und
ein Rohrleitungssegment (6), durch das das flüssige aerosolbildende Substrat von der Mikropumpe an den Zerstäuber abgegeben wird;
wobei das Heizelement ein konisches Heizelement aufweist, das sich von dem Rohrleitungssegment in einer Längsrichtung erstreckt, und der Zerstäuber zum Erwärmen des abgegebenen flüssigen aerosolbildenden Substrats in dem inneren Durchgang auf eine Temperatur ausgelegt ist, die ausreicht, um zumindest einen Teil des abgegebenen flüssigen aerosolbildenden Substrats zu verflüchtigen.

2. Aerosolerzeugungssystem nach Anspruch 1, wobei die Mikropumpe (5) ausgelegt ist, eine bestimmte Menge des flüssigen aerosolbildenden Substrats von dem Flüssigspeicherteil (3) an den inneren Durchgang des Heizelements bei Durchführung eines Pumpzyklus abzugeben.

3. Aerosolerzeugungssystem nach Anspruch 1 oder Anspruch 2, wobei das System ferner eine Kammer aufweist, in die das flüssige aerosolbildende Substrat abgegeben wird, und wobei das Heizelement innerhalb der Kammer einem Auslass der Mikropumpe nachgeschaltet angeordnet ist, durch die das flüssige aerosolbildende Substrat abgegeben wird.

4. Aerosolerzeugungssystem nach Anspruch 1, wobei der Zerstäuber (7) einem offenen Ende des Rohrleitungssegments (6) nachgeschaltet angeordnet ist.

5. Aerosolerzeugungssystem nach Anspruch 1 oder 4, wobei das Rohrleitungssegment (6) ein Kapillarrohr aufweist.

6. Aerosolerzeugungssystem nach Anspruch 1, 4 oder 5, wobei der Zerstäuber (7) eine Heizspule aufweist, die sich um einen Abschnitt des Rohrleitungssegments (6) erstreckt.

7. Aerosolerzeugungssystem nach Anspruch 1, 4 oder 5, wobei der Zerstäuber (7) eine Heizspule aufweist, die sich von dem Rohrleitungssegment (6) in Längsrichtung erstreckt.

8. Aerosolerzeugungssystem nach einem der Ansprüche 1 bis 7, wobei das Flüssigspeicherteil (3) einen starren Behälter und ein Einwegventil (18) aufweist, das ausgelegt ist, Luft in den Behälter einzulassen, wenn ein flüssiges aerosolbildendes Substrat von dem Flüssigspeicherteil an den inneren Durchgang des Heizelements abgegeben wird.

9. Aerosolerzeugungssystem nach einem der Ansprüche 1 bis 7, wobei das Flüssigspeicherteil einen flexiblen Behälter mit zumindest einer beweglichen Wand aufweist, die ausgelegt ist, das Volumen des Flüssigspeicherteils beim Abgeben eines flüssigen aerosolbildenden Substrats von dem Flüssigspeicherteil an den inneren Durchgang des Heizelements zu reduzieren.

10. Aerosolerzeugungssystem nach einem der Ansprüche 1 bis 9, wobei das Flüssigspeicherteil (3) ein Septum (19) zum Nachfüllen des Flüssigspeicherteils mit einem flüssigen aerosolbildenden Substrat aufweist.

11. Aerosolerzeugungssystem nach einem der Ansprüche 1 bis 10, wobei das Flüssigspeicherteil (3) ein Einwegventil (18) aufweist, das zwischen dem Flüssigspeicherteil und der Mikropumpe (5) angeordnet ist.

12. Aerosolerzeugungssystem nach einem der Ansprüche 1 bis 11, wobei die Strömungsgeschwindigkeit des abgegebenen flüssigen aerosolbildenden Substrats durch die Mikropumpe (5) innerhalb von 0,5 bis 2 Mikroliter pro Sekunde liegt.

13. Aerosolerzeugungssystem nach einem der Ansprüche 1 bis 12, wobei das System eine Haupteinheit und eine Patrone aufweist, wobei die Patrone mit der Haupteinheit lösbar gekoppelt ist, wobei die Haupteinheit eine Energieversorgung (1) aufweist, wobei das Flüssigspeicherteil (3) in der Patrone vorgesehen ist und wobei die Mikropumpe (5) in der Haupteinheit vorgesehen ist.

14. Verfahren zum Erzeugen eines Aerosols; aufweisend:
Speichern von flüssigem aerosolbildendem Substrat in einem Flüssigspeicherteil (3);
Abgeben, durch eine Mikropumpe (5), eines flüssigen aerosolbildenden Substrats von dem Flüssigspeicherteil durch ein Rohrleitungssegment (6) an einen inneren Durchgang innerhalb eines Heizelements eines Zerstäubers (7), wobei das Heizelement ein konisches Heizelement aufweist, das sich von dem Rohrleitungssegment in einer Längsrichtung erstreckt; und
Erwärmen, durch den Zerstäuber, des abgegebenen flüssigen aerosolbildenden Substrats in dem inneren Durchgang auf eine Temperatur, die ausreicht, um zumindest einen Teil des abgegebenen flüssigen aerosolbildenden Substrats zu verflüchtigen.

## Revendications

1. Système de génération d'aérosol, le système comprenant :
une partie de stockage de liquide (3) pour stocker un substrat formant aérosol liquide ;
un vaporisateur (7) comprenant un élément de chauffage ayant une structure définissant un passage interne ouvert ;
une micropompe (5) configurée pour fournir un substrat formant aérosol liquide depuis la partie de stockage de liquide vers le passage interne de l'élément de chauffage ; et
un segment de tubulure (6) à travers lequel le substrat formant aérosol liquide est fourni depuis la micropompe vers le vaporisateur ;
dans lequel l'élément de chauffage comprend un élément de chauffage conique s'étendant depuis le segment de tubulure dans une direction longitudinale et le vaporisateur est configuré pour chauffer le substrat formant aérosol liquide fourni au niveau du passage interne jusqu'à une température suffisante pour volatiliser au moins une partie du substrat formant aérosol liquide fourni.

2. Système de génération d'aérosol selon la revendication 1, dans lequel la micropompe (5) est configurée pour fournir une quantité déterminée de substrat formant aérosol liquide depuis la partie de stockage de liquide (3) vers le passage interne de l'élément de chauffage lors de la réalisation d'un cycle de pompage.

3. Système de génération d'aérosol selon la revendication 1 ou la revendication 2, dans lequel le système comprend en outre une chambre dans laquelle le substrat formant aérosol liquide est fourni, et dans lequel l'élément de chauffage est disposé à l'intérieur de la chambre en aval d'une sortie de la micropompe à travers laquelle le substrat formant aérosol liquide est fourni.

4. Système de génération d'aérosol selon la revendication 1, dans lequel le vaporisateur (7) est disposé en aval d'une extrémité ouverte du segment de tubulure (6).

5. Système de génération d'aérosol selon la revendication 1 ou 4, dans lequel le segment de tubulure (6) comprend un tube capillaire.

6. Système de génération d'aérosol selon la revendication 1, 4 ou 5, dans lequel le vaporisateur (7) comprend une résistance chauffante s'étendant autour d'une partie du segment de tubulure (6) .

7. Système de génération d'aérosol selon la revendication 1, 4 ou 5, dans lequel le vaporisateur (7) comprend une résistance chauffante s'étendant depuis le segment de tubulure (6) dans la direction longitudinale.

8. Système de génération d'aérosol selon l'une quelconque des revendications 1 à 7, dans lequel la partie de stockage de liquide (3) comprend un récipient rigide et un clapet unidirectionnel (18) qui est configuré pour laisser de l'air dans le récipient lors de la fourniture d'un substrat formant aérosol liquide depuis la partie de stockage de liquide vers le passage interne de l'élément de chauffage.

9. Système de génération d'aérosol selon l'une quelconque des revendications 1 à 7, dans lequel la partie de stockage de liquide comprend un récipient souple avec au moins une paroi mobile qui est configurée pour réduire le volume de la partie de stockage de liquide lors de la fourniture de substrat formant aérosol liquide depuis la partie de stockage de liquide vers le passage interne de l'élément de chauffage.

10. Système de génération d'aérosol selon l'une quelconque des revendications 1 à 9, dans lequel la partie de stockage de liquide (3) comprend un septum (19) pour recharger la partie de stockage de liquide avec du substrat formant aérosol liquide.

11. Système de génération d'aérosol selon l'une quelconque des revendications 1 à 10, dans lequel la partie de stockage de liquide (3) comprend un clapet unidirectionnel (18) disposé entre la partie de stockage de liquide et la micropompe (5).

12. Système de génération d'aérosol selon l'une quelconque des revendications 1 à 11, dans lequel le débit du substrat formant aérosol liquide fourni à travers la micropompe (5) est compris entre 0,5 et 2 microlitres par seconde.

13. Système de génération d'aérosol selon l'une quelconque des revendications 1 à 12, dans lequel le système comprend une unité principale et une cartouche, dans lequel la cartouche couplée d'une manière amovible à l'unité principale, dans lequel l'unité principale comprend une alimentation électrique (1), dans lequel la partie de stockage de liquide (3) est fournie dans la cartouche, et dans lequel la micropompe (5) est fournie dans l'unité principale.

14. Procédé de génération d'aérosol, comprenant :
le stockage de substrat formant aérosol liquide dans une partie de stockage de liquide (3) ;
la fourniture, par une micropompe (5), de substrat formant aérosol liquide à partir de la partie de stockage de liquide, à travers un segment de tubulure (6), vers un passage interne au sein d'un élément de chauffage d'un vaporisateur (7), l'élément de chauffage comprenant un élément de chauffage conique s'étendant à partir du segment de tubulure dans une direction longitudinale ; et
le chauffage, par le vaporisateur, du substrat formant aérosol liquide fourni dans le passage interne jusqu'à une température suffisante pour volatiliser au moins une partie du substrat formant aérosol liquide fourni.
